# EUROPEAN PATENT APPLICATION

(11) **EP 2 277 564 A2**
(43) Date of publication of application: **26.01.2011**
(21) Application number: 10168486.8
(22) Date of filing: 05.07.2010
(51) Int. Cl.: A61L 31/16, A61L 33/10

(54) **An anti-thrombotic coating for a medical device**

(30) Priority: 20.07.2009 US 505869
(71) Applicant: Cordis Corporation, Miami Lakes, Florida 33014 (US)
(72) Inventor: Zhao, Jonathon, Belle Mead, NJ 08502 (US); Falotico, Robert, Belle Mead, NJ 08502 (US)
(74) Representative: Belcher, Simon James

(57) **Abstract**

A conjugate between an anti-thrombotic agent and bioabsorbable polymer includes first or sub-layer which is prepared by mixing polymeric material and a biologically active agent with a solvent, thereby forming a homogeneous solution. A second or outer layer comprises an anti-thrombotic heparin-bioabsorbable polymer conjugate. This coating may be applied over inner drug-containing layers using, for example, dip coating or spray coating processes. After drying, the anti-thrombotic heparin bioabsorbable polymer conjugate remains in the outer layer of coating, allowing agent from the inner layer to elute through the coating. The outmost layer prevents thrombosis, and can modulate release kinetics of agcnt(s) contained within inner laycr(s) of the coating.

## Description

The present invention relates to a coating material for application to at least a portion of one surface of a medical device. In particular, this invention relates to anti-thrombotic and antirestenotic coating compositions having a multi-layered coating, in which the first or inner layer is formed from a polymer and one or more biologically active agents, and a second or outer layer comprises an anti-thrombotic heparin-bioabsorbable polymer conjugate. This invention also relates to methods of making a heparin-bioabsorbable polymer conjugate and applying a coating material comprising such an anti-thrombotic heparin-bioabsorbable polymer conjugate over at least a portion of the surface of an implantable medical device.

Atherogenic arterial narrowing (stenosis), and the gradual narrowing of a blood vessel following an angioplasty procedure or a stent implantation (restenosis) are tow commonly encountered vascular diseases. Stenosis refers to the narrowing or constriction of a vessel, which is usually due to the buildup of fat, cholesterol, and other substances over time. In severe cases, stenosis can completely clog a vessel. Thrombosis refers to the formation of blood clots on or near an implanted device in the blood vessel. The clot is usually formed by an aggregation of blood factors, primarily platelets and fibrin, with entrapment of cellular elements. Thrombosis, like stenosis, frequently causes vascular obstruction at the point of its formation. Both restenosis and thrombosis are two serious and potentially fatal conditions that need medical intervention.

One approach to clearing an artery that has been constricted or clogged due to stenosis is percutaneous transluminal coronary angioplasty (PTCA) or balloon coronary angioplasty. In this procedure, a balloon catheter is inserted and expanded in the constricted portion of the vessel for clearing the blockage. About one-third of patients who undergo PTCA suffer from restenosis, the re-narrowing of the widened segment, within about six months of the procedure. Restenosed arteries may have to undergo another angioplasty.

Restenosis can be inhibited by a common procedure that consists of inserting a stent into the effected region of the artery instead of, or along with, angioplasty. A stent is a tube made of metal or plastic, which can have either solid walls or mesh walls. Most stents in use are metallic and are either self-expanding or balloon-expandable. The decision to undergo a stent insertion procedure depends on certain features of the arterial stenosis. These include the size of the artery and the location of the stenosis. The function of the stent is to buttress the artery that has recently been widened using angioplasty, or, if no angioplasty was used, the stent is used to prevent elastic recoil of the artery. Stents are typically implanted via a catheter. In the case of a balloon-expandable stent, the stent is collapsed to a small diameter and slid over a balloon catheter. The catheter is then manoeuvred through the patient's vasculature to the site of the lesion or the area that was recently widened. Once in position, the stent is expanded and locked in place. The stent stays in the artery permanently, holds it open, improves blood flow through the artery, and relieves symptoms (usually chest pain).

Stents are not completely effective in preventing restenosis at the implant site. Restenosis can occur over the length of the stent and/or past the ends of the stent. Physicians have recently employed new types of stents that are coated with a thin polymer film loaded with a drug that inhibits smooth cell proliferation. The coating is applied to the stent prior to insertion into the artery using methods well known in the art, such as a solvent evaporation technique. The solvent evaporation technique entails mixing the polymer and drug in a solvent. The solution comprising polymer, drug, and solvent can then be applied to the surface of the stent by either dipping or spraying. The stent is then subjected to a drying process, during which the solvent is evaporated, and the polymeric material, with the drug dispersed therein, forms a thin film layer on the stent.

The release mechanism of the drug from the polymeric materials depends on the nature of the polymeric material and the drug to be incorporated. The drug diffuses through the polymer to the polymer-fluid interface and then into the fluid. Release can also occur through degradation of the polymeric material. The degradation of the polymeric material may occur through hydrolysis or an enzymatic digestion process, leading to the release of the incorporated drug into the surrounding tissue.

An important consideration in using coated stents is the release rate of the drug from the coating. It is desirable that an effective therapeutic amount of the drug be released from the stent for a reasonably long period of time to cover the duration of the biological processes following and an angioplasty procedure or the implantation of a stent. Burst release, a high release rate immediately following implantation, is undesirable and a persistent problem. While typically not harmful to the patient, a burst release "wastes" the limited supply of the drug by releasing several times the effective amount required and shortens the duration of the release period. Several techniques have been developed in an attempt to reduce burst release. For example, US-6258121 discloses a method of altering the release rate by blending two polymers with differing release rates and incorporating them into a single layer.

Another potential problem associated with the implantation of a drug eluting stent is thrombosis that may occur at different times following the implantation of a stent. A thrombus formation on the surface of a stent is frequently lethal, leading to a high mortality rate of between 20 to 40% in the patients suffering from a thrombosis in a vessel.

One way to address the formation of stent thrombosis is through the use of a potent anticoagulant such as a heparin. Heparin is a substance that is well known for its anticoagulation ability. It is known in the art to apply a thin polymer coating loaded with heparin onto the surface of a stent using the solvent evaporation technique. For example, US-5837313 discloses a method of preparing a heparin coating composition. Unfortunately heparin because of its hydrophilic nature elutes out of the polymer matrix quickly without staying on the surface of the implant where the thrombosis occurs. The leaching of a heparin molecule and the infiltration of water into the polymer matrix where an anti-restenotic agent is contained may cause a rapid elution of the drug from the polymer matrix and consequently a less than desirable efficacy of the drug. The stability of the drug may also be adversely affected by the presence of water.

Therapeutic or biologically active agents, such as those used to prevent thrombosis, are included within a coating whereby after implantation of the device, the therapeutic agent will be eluted from the coating to the surrounding tissue of the body. Thus, the coating must allow the pharmaceutical or therapeutic agents to permeate there through. It is also desirable that the coating functions as a physical barrier, a chemical barrier, or a combination thereof to control the elution of the pharmaceutical or therapeutic agents from the underlying basecoat. This is accomplished by controlling the access of water and other fluids to the therapeutic agent. Absent regulation of fluid flow, the agent will elute more rapidly than desired. For example, if it is desired to have the agent released within a month, rapid hydration may lead to the agent being released within days.

Although effective in reducing restenosis, some of the components of the coatings used for a drug-eluting stent may increase the risk of thrombosis. Drug eluting stents are typically not associated with an increase of acute and subacute thrombosis (SAT), or a medium term thrombosis (30 days after stent implantation) following a stent implantation. Long term clinical follow up studies, however, suggest that these devices may be involved with increased incident rates of very long term thrombosis (LST). Although the increase of LST has been found to be less than 1%, a high mortality rate is usually associated with LST. One way to prevent this is to include a coating of an anti-coagulant, such as heparin, on the device.

Few devices can deliver an agent to prevent restenosis while also ensuring that the coatings that the agent is embedded in will not contribute to thrombosis. One solution can involve combining the agent with an anti-coagulant within a coating, however this fails due to the hydrophilic nature of anti-coagulants. For example, therapeutic agent is embedded in the matrix of a polymer coating by solvent processing. If an anti-coagulant is also embedded in the polymer matrix, it will attract water in an uncontrolled manner. This can happen during manufacturing or when the coated device is implanted and will adversely affect the stability or efficacy of the agent and/or interfere with the desired elution profile.

Nonetheless, several approaches have been proposed for combining anti-thrombotic and therapeutic agents within the coatings for an implantable medical device. US-5525348 discloses a method of complexing pharmaceutical agents (including heparin) with quaternary ammonium components or other ionic surfactants and bound with water insoluble polymers as an antithrombotic coating composition. This method suffers from the possibility of introducing naturally derived polymer such as cellulose, or a derivative thereof, which is heterogeneous in nature and may cause unwanted inflammatory reactions at the implantation site. These ionic complexes between an antithrombotic agent such as heparin and an oppositely charged carrier polymer may also negatively affect the coating integration, and if additional pharmaceutical agents are present, may affect the shelf stability and release kinetics of these pharmaceutical agents.

A slightly different approach is disclosed in US-6702850, US-6245753 and US-7129224, in which anti-thrombotic agents such as heparin are covalently conjugated to a non-absorbable polymer such as a polyacrylic acid before use in a coating formulation. The overall hydrophobicity of these conjugates is further adjusted by addition of a hydrophobic agent such as octadecylamine, which is an amine with a long hydrocarbon chain. This approach has several potential disadvantages such as the known toxicity of polyacrylic acid after heparin is metabolized in vivo. The addition of a hydrophobic amine also raises the concern of tissue compatibility and reproduction of the substitution reactions of each step. Moreover, the remaining components of the coating are not biodegradable.

Another approach to increase the solubility and potentially miscibility of the heparin and its derivatives is through making a pro-drug of heparin that may have a higher solubility in an organic solvent and be more amenable to coating processes that are commonly used in surface modification and drug elution medical device making. For instance, US-7396541 discloses methods of making a heparin pro-drug. The heparin product can be a conjugate between a heparin and modified heparin species that contain specific functional groups such as -CHO (aldehyde) and a drug such as everolimus, or a polymer such poly(lysine). A basic requirement for such scheme is the utilization of carboxyl or -CHO groups in the heparin. However, this approach suffers from certain drawbacks, including that the reaction is complex and additional chemical transformations may be involved to prepare special heparin derivatives, and that there is non-specificity in reaction and a lack of control for the conversion degree of percentage. In the end, when the conjugate is degraded the specific activity of the drug, the heparin molecules, and the polymers may be lost since the degradation is non-specific.

Another anti-thrombotic coating approach is disclosed in US-6559132, US-6461665 and US-6767405 in which a carrier molecule such as chitosan is conjugated to an activated metal surface of a medical device. Heparin is then covalently conjugated to an intermediate molecule. This process may be repeated several times until a desired anti-thrombotic layer is achieved. Alternatively, this coating can be achieved in a batch process mode. This approach, however, is not readily applicable to a medical device that is coated with a polymer coating that contains one or more pharmaceutical agents. Some of these successful anti-restenotic agents such as sirolimus may be damaged during these conjugating processes, especially these processes where aqueous processes are involved.

WO-2005/097223 discloses a method in which a mixture is applied in solution which contains heparin conjugated with photoactive crosslinking agents, and other durable polymers such as poly(butyl methacrylate) and poly(vinyl pyrrolidone). The mixture is crosslinked with UV light in the solution or after the coating is applied. The potential disadvantage of this approach is that the incorporated drug (or drugs) may be adversely affected by the high energy UV light during crosslinking process, or worse, the drugs may be crosslinked to the matrix polymers if they possess functional groups that may be activated by the UV energy.

An approach which is disclosed in US-2005/0191333, US-2006/0204533 and WO-2006/099514 uses a low molecular weight complex of heparin and a counter ion (stearylkonium heparin), or a high molecular weight polyelectrolyte complex , such as dextran, pectin to form a complex form of an anti-thrombotic entity. These anti-thrombotic complexes are further dispersed in a polymer matrix which may further comprise a drug. Such approaches create a heterogeneous matrix of a drug and a hydrophilic species of heparin in which the hydrophilic species attract water before and after the implantation to adverse the stability and release kinetics of the drug. In addition, the desired anti-thrombotic functions of heparin and similar agent should be preferably located on the surface, not being eluted away from the surface of a coated medical device.

There remains a need for a coating material that can satisfy the requirements described above for applying on at least one surface of a medical device and can be prepared through a process that is compatible with the sensitive pharmaceutical or therapeutic agents impregnated in the coatings. This helps to fill a need for a coating that treats both restenosis and prevents thrombosis when applied to the outer surface of a stent.

The present invention provides a conjugate between a heparin and a bioabsorbable polymer with a free carboxyl end group. In addition, a method is provided for applying a coating comprising a heparin bioabsorbable polymer conjugate to at least a portion of an implantable device to prevent or reduce the formation of thrombosis on the surface of the device. The outmost layer of the coating comprises a conjugate which prevents the formation of thrombosis, and also serves to modulate the release kinetics of the agent(s) contained within an inner layer(s) of the coating.

A first or sub-layer of the coating is prepared by mixing a polymeric material and a biologically active agent with a solvent, thereby forming a homogeneous solution. The polymeric material can be selected from a wide range of synthetic materials, but a poly(lactide-co-glycolide) (PLGA) can be preferred. The biologically active agent is selected depending on the desired therapeutic results. For example, an antiproliferative drug such as paclitaxel, an immunosuppressant, such as a rapamycin, and/or anti-inflammatory drug, such as dexamethasone, may be included in the inner layer. Once prepared, the solution can be applied to the device through a dipping or spraying process. During drying, the solvent evaporates, and a thin layer of the polymeric material loaded with the biologically active agent is left coated over the stent. The coating is not limited to just one inner layer or biologically active agent per layer. It is contemplated that one or more distinct biologically active agents may be included in each layer or that more than one inner layer may be loaded with a biologically active agent, or both.

The second or outer layer comprises an anti-thrombotic heparin-bioabsorbable polymer conjugate. This coating may be applied over the inner drug-containing layers using, for example, a dip coating or spray coating process. The outer layer can comprise an anti-thrombotic heparin-bioabsorbable polymer conjugate which may be dissolved in a mixed solvent system comprising ethyl acetate (EA) and isopropanol (IPA). The solution is then sprayed onto the surface of the device that has already been coated with the agent-containing layer as described above. After drying, the anti-thrombotic heparin bioabsorbable polymer conjugate remains in the outer layer of the coating, allowing agent from the inner layer to elute through the coating.

The coated device is inserted into an afflicted area of a body, for example, a vessel like the coronary artery, using an appropriate procedure that depends on the properties of the device. Once in place, the device will hold the vessel open. The biologically active agent will be released from the first layer, thereby providing the desired therapeutic result, such as inhibiting smooth cell proliferation. The anti-thrombotic heparin-bioabsorbable polymer conjugate in the outmost layer becomes partially hydrated and prevents blood coagulation on and around the device, thus inhibiting thrombosis and subacute device thrombosis. In addition, the anti-thrombotic heparin-bioabsorbable polymer conjugate in the outmost layer may additionally reduce or prevent the burst release of the biologically active agent from the inner drug containing layer, thereby allowing the release to occur over a relatively extended period of time.

One or more layers of polymeric compositions are applied to a medical device to provide a coating thereto. The polymeric compositions perform differing functions. For example, one layer may comprise a base coat that allows additional layers to adhere thereto. An additional layer(s) can carry bioactive agents within their polymer matrices. Alternatively, a single coat may be applied in which the polymeric composition is such that the coat performs multiple functions, such as allowing the coating to adhere to the device and housing an agent that prevents thrombosis. Other functions include housing an agent to prevent restenosis. Often, however, the chemical requirement of each agent limits the number of agents a coating may carry. For example, an antithrombotic agent tends to be hydrophilic while an anti-proliferative agent tends to be comparatively hydrophobic. Hence, it is desired to entrap a hydrophobic agent within the matrix of a polymer coating to limit its exposure to water and control its elution from the matrix. The present invention maintains two agents having differing properties in close proximity by providing a conjugate between a heparin and a bioabsorbable polymer with a free carboxyl end group. When coated onto a medical device the conjugate ensures that the anti-thrombotic agent is substantially oriented away from any hydrophobic agents that may be contained within the polymer matrix.

The following definitions are provided for ease of understanding the invention:
The term "stent" means a generally tubular structure constructed from any biocompatible material that is inserted into a conduit to keep the lumen open and prevent closure due to a stricture or external compression.
The term stent "biologically active agent" means a drug or other substance that has therapeutic value to a living organism including without limitation antithrombotics, anticancer agents, anticoagulants, antiplatelet agents, thrombolytics, antiproliferatives, antiinflammatories, anti-restenotics, agents that inhibit restenosis, smooth muscle cell inhibitors, antibiotics, and the like, and/or mixtures thereof and/or any substance that may assist another substance in performing the function of providing therapeutic value to a living organism.
The term "polymer" means a macromolecule made of repeating monomer units or co-monomer units.
The term "macromolecule" means synthetic macromolecules, proteins, biopolymers and other molecules with a molecular weight typically greater than 1000.
The term "effective amount" means an amount of pharmacologically active agent that is nontoxic but sufficient to provide the desired local or systemic effect and performance at a reasonable benefit/risk ratio attending any medical treatment.

Exemplary anticancer drugs include acivicin, aclarubicin, acodazole, acronycine, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus calmette-guerin (BCG), Baker's Antifol (soluble), beta-2'-deoxythioguanosine, bisantrene hydrochloride, bleomycin sulphate, busulphan, buthionine sulphoximine, ceracemide, carbetimer, carboplatin, carmustine, chlorambucil, chloroquinoxaline-sulphonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HCl, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, edatrexate, edelfosine, eflomithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5-fluorouracil, Fluosol, flutamide, gallium nitrate, gemcitabine, goserelin acetate, hepsulfam, hexamethylene bisacetamide, homoharringtonine, hydrazine sulphate, 4-hydroxyandrostenedione, hydrozyurea, idarubicin hydrochoride, ifosfamide, interferon alfa, interferon beta, interferon gamma, interleukin-1 alpha and beta, interleukin-3, interleukin-4, interleukin-6, 4-ipomeanol, iproplatin, isotretinoin, leucovorin calcium, leuprolide acetate, levamisole, liposomal daunorubicin, liposome encapsulated doxorubicin, lomustine, lonidamine, maytansine, mechlorethamine hydrochloride, melphalan, menogaril, merbarone, 6-mercaptopurine, mesna, methanol extraction residue of Bacillus calmette-guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, tumor necrosis factor, uracil mustard, vinblastine sulphate, vincristine sulphate, vindesine, vinorelbine, vinzolidine, Yoshi 864, zorubicin, and mixtures thereof.

Exemplary anti-inflammatory drugs include classic non-steroidal anti-inflammatory drugs (NSAIDS), such as aspirin, diclofenac, indomethacin, sulindac, ketoprofen, flurbiprofen, ibuprofen, naproxen, piroxicam, tenoxicam, tolmetin, ketorolac, oxaprosin, mefenamic acid, fenoprofen, nambumetone (relafen), acetaminophen (sold under the trade mark Tylenol), and mixtures thereof; COX-2 inhibitors, such as nimesulide, NS-398, flosulid, L-745337, celecoxib, rofecoxib, SC-57666, DuP-697, parecoxib sodium, JTE-522, valdecoxib, SC-58125, etoricoxib, RS-57067, L-748780, L-761066, APHS, etodolac, meloxicam, S-2474, and mixtures thereof; glucocorticoids, such as hydrocortisone, cortisone, prednisone, prednisolone, methylprednisolone, meprednisone, triamcinolone, paramethasone, fluprednisolone, betamethasone, dexamethasone, fludrocortisone, desoxycorticosterone, and mixtures thereof.

Exemplary anti-restenosis drugs include a rapamycin or its various derivatives and analogs. The most important rapamycin drugs include sirolimus, everolimus, biolimus, zotarolimus and CCI-779. Rapamycin is a macroyclic triene antibiotic produced by streptomyces hygroscopicus as disclosed in US-3929992. It has been found that rapamycin inhibits the proliferation of vascular smooth muscle cells *in vivo.* Accordingly, rapamycin may be utilized in treating intimal smooth muscle cell hyperplasia, restenosis and vascular occlusion in a mammal, particularly following either biologically or mechanically mediated vascular injury, or under conditions that would predispose a mammal to suffering such a vascular injury. Rapamycin functions to inhibit smooth muscle cell proliferation and does not interfere with the re-endothelialization of the vessel walls.

Rapamycin functions to inhibit smooth muscle cell proliferation through a number of mechanisms. In addition, rapamycin reduces the other effects caused by vascular injury, for example, inflammation. The operation and various functions of rapamycin are described in detail below. Rapamycin as used throughout this application shall include rapamycin, rapamycin analogs, derivatives and congeners that bind FKBP12 and possess the same pharmacologic properties as rapamycin.

Rapamycin reduces vascular hyperplasia by antagonizing smooth muscle proliferation in response to mitogenic signals that are released during angioplasty. Inhibition of growth factor and cytokine mediated smooth muscle proliferation at the late G1 phase of the cell cycle is believed to be the dominant mechanism of action of rapamycin. However, rapamycin is also known to prevent T-cell proliferation and differentiation when administered systemically. This is the basis for its immunosuppresive activity and its ability to prevent graft rejection.

The molecular events that are responsible for the actions of rapamycin, a known anti-proliferative, which acts to reduce the magnitude and duration of neointimal hyperplasia, are still being elucidated. It is known, however, that rapamycin enters cells and binds to a high-affinity cytosolic protein called FKBP12. The complex of rapamycin and FKPB 12 in turn binds to and inhibits a phosphoinositide (PI)-3 kinase called the "mammalian Target of Rapamycin" or mTOR. The mammalian Target of Rapamycin is a protein kinase that plays a key role in mediating the downstream signaling events associated with mitogenic growth factors and cytokines in smooth muscle cells and T lymphocytes. These events include phosphorylation of p27, phosphorylation of p70 s6 kinase and phosphorylation of 4BP-1, an important regulator of protein translation.

In an embodiment of the present invention, a first or inner layer of a coating comprises a polymeric film loaded with a biologically active agent that prevents smooth cell proliferation and migration, such as a rapamycin. One manner in which the agent is placed within the matrix of the polymer involves using a solvent or mixture of solvents whereby the agent and polymer are dissolved therein. As the mixture dries, the solvent is removed leaving the agent entrapped within the matrix of the polymer. Examples of polymers that can be used for making the inner/ first polymeric layer include polyurethanes, polyethylene terephthalate (PET), PLLA-poly-glycolic acid (PGA) copolymer (PLGA), polycaprolactone (PCL) poly-(hydroxybutyrate-co-hydroxyvalerate) copolymer (PHBV), poly(vinylpyrrolidone) (PVP), polytetrafluoroethylene (PTFE), poly(2-hydroxyethylmethacrylate) (poly-HEMA), poly(etherurethane urea), silicones, acrylics, epoxides, polyesters, urethanes, polyphosphazene polymers, fluoropolymers, polyamides, polyolefins, and mixtures thereof. Exemplary bioabsorbable polymers that can be used for making the inner/ first polymeric film include polycaprolactone (PCL), poly-D, L-lactic acid (DL-PLA), poly-L-lactic acid (L-PLA), poly(hydroxybutyrate), polydioxanone, polyorthoester, polyanhydride, poly(glycolic acid), polyphosphoester, poly (amino acids), poly(trimethylene carbonate), poly(iminocarbonate), polyalkylene oxalates, polyphosphazenes, and aliphatic polycarbonates.

The second or outmost layer may comprise an anti-thrombotic heparin-bioabsorbable polymer conjugate with strong anticoagulation properties. The second layer of anti-thrombotic heparin-bioabsorbable polymer conjugate may additionally have the effect of preventing a burst release of the biologically active agent dispersed in the first or inner layer, resulting in a relatively longer release period of the biologically active agent. The first layer may contain more than one biologically active agent.

The coating can be applied to a medical device such as a stent or a stent-graft. Any substrate, medical device, or part thereof having contact with organic fluid, or the like, may also be coated with the coating. For example, other devices such as vena cava filters and anastomosis devices may be used with coatings having agents therein or the devices themselves may be fabricated with polymeric materials that have the drugs contained therein. Any of the stents or other medical devices described herein may be used for local or regional drug delivery. Balloon expandable stents may be utilized in any number of vessels or conduits, and are particularly well suited for use in coronary arteries. Self expanding stents, on the other hand, are particularly well suited for use in vessels where crush recovery is a critical factor, for example, in the carotid artery.

In general, a metal stent, such as those manufactured from stainless steel, cobalt chromium alloys, but plastic or other appropriate materials may be used, however, the coating may also be applied to a polymeric stent. In one embodiment, the stent is a L605 cobalt chromium alloy. It is desirable, but not required, that the first and second coatings cover at least a portion of the entire stent surface. The application of the first layer is accomplished through a solvent evaporation process or some other known method. The solvent evaporation process entails combining the polymeric material and the biologically active agent with a solvent, such as tetrahydrofuran (THF), which are then mixed by stirring to form a mixture. An example of a polymeric material which can be used for the first layer comprises polyurethane and an example of a biologically active agent comprises a rapamycin. The mixture is then applied to the surface of the stent by either: (1) spraying the solution onto the stent; or (2) dipping the stent into the solution. After the mixture has been applied, the stent is subjected to a drying process, during which, the solvent evaporates and the polymeric material and biologically active agent form a thin film on the stent. Alternatively, a plurality of biologically actives agent can be added to the first layer.

The second or outmost layer of the stent coating comprises an anti-thrombotic heparin-bioabsorbable polymer conjugate. The anti-thrombotic heparin-bioabsorbable polymer conjugate may be soluble in organic solvents or mixtures of organic solvents of varying polarity. The heparin may comprise an unfractionated heparin, fractionated heparin, a low molecular weight heparin, a desulphated heparin and heparins of various mammalian sources. Exemplary anti-thrombotic agents may include: Vitamin K antagonist such as Acenocoumarol, Clorindione, Dicumarol (Dicoumarol), Diphenadione, Ethyl biscoumacetate, Phenprocoumon, Phenindione, Tioclomarol, Warfarin; Heparin group anti-platelet aggregation inhibitors such as Antithrombin III, Bemiparin, Dalteparin, Danaparoid, Enoxaparin, Heparin, Nadroparin, Parnaparin, Reviparin, Sulodexide, Tinzaparin; other platelet aggregation inhibitors such as Abciximab, Acetylsalicylic acid (Aspirin), Aloxiprin, Beraprost, Ditazole, Carbasalate calcium, Cloricromen, Clopidogrel, Dipyridamole, Eptifibatide, Indobufen, Iloprost, Picotamide, Prasugrel, Prostacyclin, Ticlopidine, Tirofiban, Treprostinil, Triflusal; enzymatic anticoagulants such as Alteplase, Ancrod, Anistreplase, Brinase, Drotrecogin alfa, Fibrinolysin, Protein C, Reteplase, Saruplase, Streptokinase, Tenecteplase, Urokinase; direct thrombin inhibitors such as Argatroban, Bivalirudin, Dabigatran, Desirudin, Hirudin, Lepirudin, Melagatran, Ximelagatran; and other antithrombotics such as Dabigatran, Defibrotide, Dermatan sulphate, Fondaparinux, Rivaroxaban.

In an exemplary embodiment, the anti-thrombotic heparin-bioabsorbable polymer conjugate is prepared as follows. First, a cyclic dimer of d,1-lactide, is polymerized at elevated temperature of about 140°C, in the presence of a stannous octoate catalyst (Sn(OCt)₂) and a predetermined amount of water as the ring opening initiator. Ring opening polymerization results in an end product that contains a homopolymer of polyester. The molecular weight of each polymer is determined by the ratio between the cyclic dimer and the initiator. The higher the ratio between the cyclic dimer to the initiator, the higher the molecular weight of the polymer. The initiator used in ring opening polymerization determines the end groups of the polymerized polyester. A monofunctional initiator such as ethanol will lead to a final polymer with only one hydroxyl group in the end. A di-functional initiator, such as ethylene glycol, will lead to a polymer with hydroxyl groups on both ends.

In one embodiment of the present invention an initiator, such as water, creates a carboxyl group at one end of the final polymer that may be further, and easily, employed in the subsequent conjugation reaction with a heparin molecule. The bioabsorbable polymer with a carboxyl end group synthesized in the fist step, may be activated by using N,N-dicyclohexylcarbodiimide hydrochloride (DDC) and N,N-hydroxysuccinimide (NHS) before the coupling reaction with the amine groups or hydroxyl groups of a heparin molecule. Although any heparin molecule, a recombinant heparin, heparin derivatives or heparin analogues (having a preferred weight of 1,000 to 10⁶ Daltons) may be used in the coupling reaction to make the final anti-thrombotic heparin-bioabsorbable polymer conjugate, it is preferred to use a desulphated heparin to increase the coupling efficiency of the reaction.

Once the anti-thrombotic heparin-bioabsorbable polymer conjugate is prepared, the second layer comprising the anti-thrombotic heparin polymer conjugate may be applied directly over the first layer using the solvent evaporation method or other appropriate method. After the solvent has evaporated from the surface of an implantable medical device, a thin film of comprising anti-thrombotic heparin-bioabsorbable polymer conjugate is formed on the outmost surface of the device.

Embodiments of the invention are described below by way of example with reference to the accompanying drawings, in which:
Figure 1 is a schematic representation of ring opening polymerization of a mixture of lactide and glycolide dimers with water as the initiator to form a carboxyl ended bioabsorbable polymer (PLGA).
Figure 2a is another schematic of a conjugation reaction of a carboxyl ended PLGA polymer and an amine group of heparin molecule.
Figure 2b is another schematic of a conjugation reaction of a carboxyl ended PLA polymer and an amine group of heparin molecule.
Figure 2c is another schematic of a conjugation reaction of a carboxyl ended PLGA polymer and a hydroxyl group of heparin molecule.
Figure 2d is another schematic of a conjugation reaction of a carboxyl ended PLA polymer and a hydroxyl group of heparin molecule.
Figure 3 is a schematic of a coating configuration applied to the surface of a medical device with the conjugate of the present invention being present in an outer layer.

The following examples illustrate the creation of the conjugate.

### Example 1

### Preparation Of A Bioabsorbable Polymer With Carboxyl End Groups

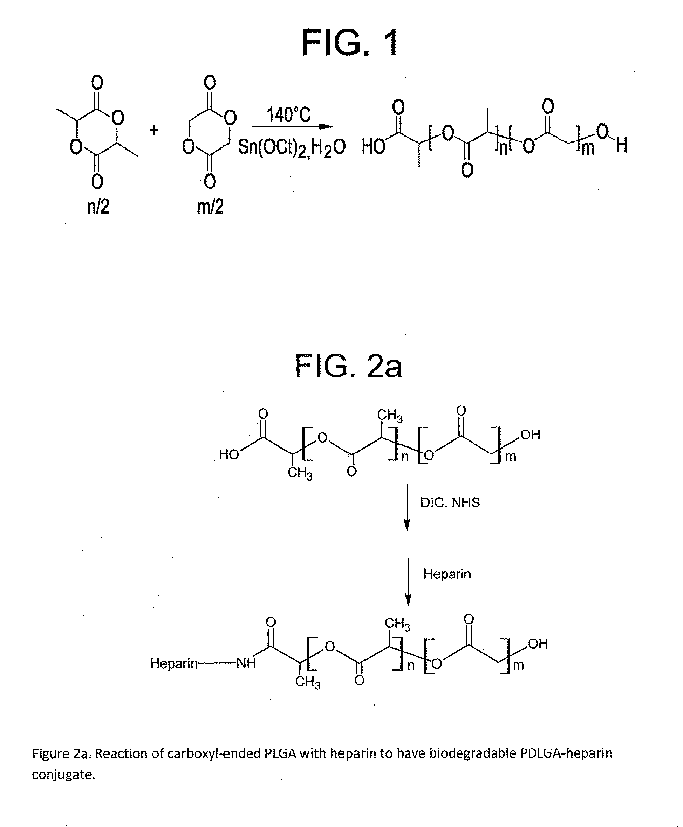

A pre-determined amount of d,1-lactide and glycolide (50:50 molar ratio, both from Purac USA) are transferred to a dried round bottom glass reactor equipped with a magnetic stir bar. A pre-determined amount of water and a toluene solution containing Stannous Octoate are added to the glass reactor. The glass reactor is then sealed with a stopper and cycled three times between an argon gas and vacuum to remove the air and oxygen inside the reactor. The sealed reactor is then gradually heated to 140°C under vacuum and kept stirred with the magnetic stir bar. Upon completion of the reaction, the polymer is dissolved in methylene chloride and precipitated in ethanol and dried under vacuum and low heat. The process is schematically illustrated in Figure 1.

Similarly d,1-lactide alone may be used in the same reaction as above, instead of the mixture of lactide and glycolide to synthesize PDLA for the conjugate.

### Example 2a

### Preparation Of Anti-Thrombotic Heparin-Bioabsorbable Polymer Conjugate

The bioabsorbable PDLGA polymer made in example 1 is dissolved in dimethylformamide (DMF), followed by dissolution ofN-hydroxylsuccinimide (NHS) and dicyclohexylcarbodiimide (DCC). The mole ratio of PDLGA, NHS, and DCC is 1:1.6 :1.6. The resulting solution is kept for 5 hours at room temperature under vacuum. The byproduct, dicyclohexylurea (DCU), and unreacted DCC and NHS are removed by filtration and extraction with water. The activated bioabsorbable polymer is then dissolved in DMF and reacted with desulphated heparin for 4 hours at room temperature. The final heparin PLGA conjugate is then precipitated and freeze dried. The process is schematically illustrated in Figure 2a.

### Example 2b

### Preparation Of Anti-Thrombotic Heparin-Bioabsorbable Polymer Conjugate

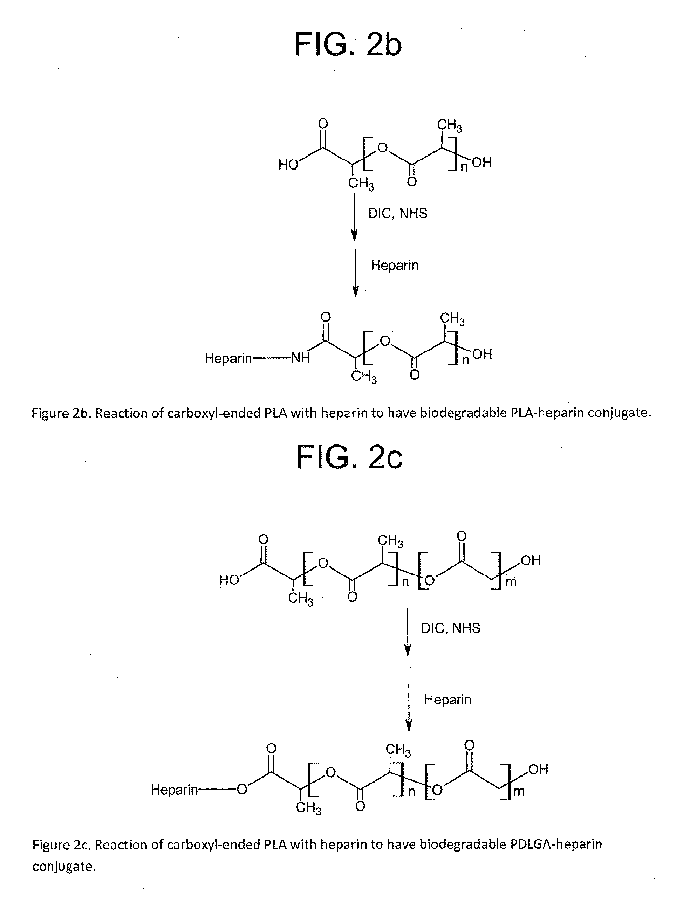

The bioabsorbable PDLA polymer made above is dissolved in dimethylformamide (DMF), followed by dissolution of N-hydroxylsuccinimide (NHS) and dicyclohexylcarbodiimide (DCC). The mole ratio of PLA, NHS, and DCC is 1:1.6:1.6. The resulting solution is kept for 5 hours at room temperature under vacuum. The byproduct, dicyclohexylurea (DCU), and unreacted DCC and NHS are removed by filtration and extraction with water. The activated bioabsorbable polymer is then dissolved in DMF and reacted with desulphated heparin for 4 hours at room temperature. The final heparin PLGA conjugate is then precipitated and freeze dried. The process is schematically illustrated in Figure 2b.

### Example 2c

### Preparation Of Anti-Thrombotic Heparin-Bioabsorbable Polymer Conjugate

The bioabsorbable PDLGA polymer made example 1 is dissolved in dimethylformamide (DMF), followed by dissolution ofN-hydroxylsuccinimide (NHS) and dicyclohexylcarbodiimide (DCC). The mole ratio of PDLGA, NHS, and DCC is 1:1.6:1.6. The resulting solution is kept for 5 hours at room temperature under vacuum. The byproduct, dicyclohexylurea (DCU), and unreacted DCC and NHS are removed by filtration and extraction with water. The activated bioabsorbable polymer is then dissolved in DMF and reacted with heparin for 4 hours at room temperature. The final heparin PLGA conjugate is then precipitated and freeze dried. The process is schematically illustrated in Figure 2c.

### Example 2d

### Preparation Of Anti-Thrombotic Heparin-Bioabsorbable Polymer Conjugate

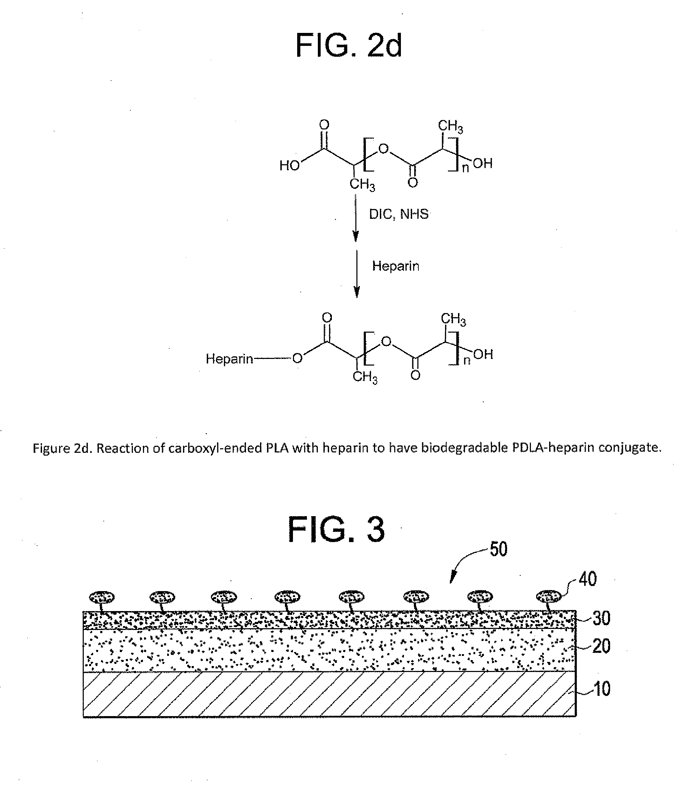

The bioabsorbable PDLA polymer made above is dissolved in dimethylformamide (DMF), followed by dissolution of N-hydroxylsuccinimide (NHS) and dicyclohexylcarbodiimide (DCC). The mole ratio of PDLA, NHS, and DCC is 1:1.6:1.6. The resulting solution is kept for 5 hours at room temperature under vacuum. The byproduct, dicyclohexylurea (DCU), and unreacted DCC and NHS are removed by filtration and extraction with water. The activated bioabsorbable polymer is then dissolved in DMF and reacted with heparin for 4 hours at room temperature. The final heparin PLGA conjugate is then precipitated and freeze dried. The process is schematically illustrated in Figure 2d.

### Example 3

Coating Of A Drug Eluting Stent With An Outmost Layer Comprising A Heparin Absorbable Polymer Conjugate

A coated medical device 50 is shown in Figure 3. The surface 10 of a cobalt chromium stent is spray coated with a drug containing polymeric solution 20, which may comprise for example, ethyl acetate (EA) containing PLGA and rapamycin. The weight ratio between PLGA and rapamycin is 2:1. After the drug-containing layer 20 is dried, a coating solution 30 containing a heparin absorbable polymer conjugate is spray coated onto the first drug-containing layer 20. After the coating solution 30 is dried, it will result in a thin film with the heparin 40 located substantially on the outermost surface.

## Claims

1. A coating comprising a conjugate of heparin and a biodegradable polymer which does not have additional pharmaceutical or biologically active functional groups, in which the biodegradable polymer is formed from at least one cyclic lactone via a ring opening polymerization.

2. The coating according to Claim 1, in which the heparin is selected from a group consisting of unmodified heparin, partially degraded heparin, low molecular weight heparin (LMWH), and desulphated heparin.

3. The coating according to Claim 1, in which the cyclic lactone is selected from a group consisting of L,L-lactides, D,L-lactides, glycolides, capralactone, trimethylene chloride (TMC), dioxanone and lactones thereof.

4. The coating according to Claim 1, in which the conjugate has the following structure: 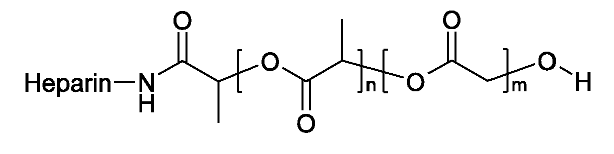 in which n and m are independently an integer of 1 to 1000.

5. The coating according to claim 1 further comprises a pharmaceutical or biologically active compound selected from the group consisting of anti-restenotic, anti-inflammatory, anti-thrombotic, and anti-proliferative compounds.

6. A medical device which has a coating applied to a surface by a process which includes at least one of spray coating, dip-coating, and ink-jetting, in which the coating is as claimed in any one of claims 1 to 5.
